(19) Europäisches Patentamt
European Patent Office
Office européen des brevets

(11) Numéro de publication : **0 594 507 A1**

(12) # DEMANDE DE BREVET EUROPEEN

(21) Numéro de dépôt : **93402581.8**

(51) Int. Cl.$^5$ : **A61K 31/165**

(22) Date de dépôt : **20.10.93**

(30) Priorité : **23.10.92 FR 9212700**

(43) Date de publication de la demande : **27.04.94 Bulletin 94/17**

(84) Etats contractants désignés : **AT BE CH DE DK ES FR GB GR IE IT LI LU MC NL PT SE**

(71) Demandeur : **LABORATOIRE L. LAFON**
**19 Avenue du Professeur Cadiot**
**F-94701 Maisons Alfort (FR)**

(72) Inventeur : **Laurent, Philippe**
**37, rue de la Glacière**
**F-69600 Oullins (FR)**

(74) Mandataire : **Le Guen, Gérard et al**
**CABINET LAVOIX 2, place d'Estienne d'Orves**
**F-75441 Paris Cédex 09 (FR)**

(54) **Utilisation du modafinil pour le traitement de l'incontinence urinaire et fécale.**

(57) La présente invention concerne l'utilisation du modafinil pour la fabrication d'un médicament pour le traitement de l'incontinence urinaire et fécale et des troubles sphinctériens urétro-vésicaux et anaux.

EP 0 594 507 A1

La présente invention concerne une nouvelle utilisation en thérapeutique du modafinil.

Le modafinil ou benzhydrylsulfinyl acétamide est un composé de formule

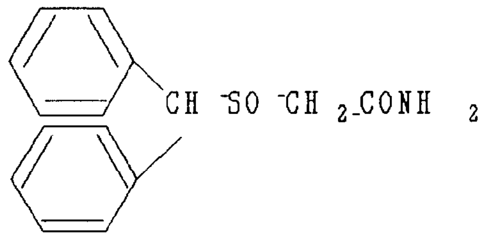

Ce composé et son utilisation thérapeutique comme agent actif sur le système nerveux central en tant que psycho-stimulant non amphétaminique ont été décrits dans le brevet FR-A-2 385 693.

On a également décrit les isomères lévogyre et dextrogyre du modafinil dans EP-A-0 233 106.

On a maintenant découvert que le modafinil et ses isomères possèdent un effet pharmacologique sur le système musculo-sphinctérien de l'appareil urinaire vésico-urétéral et sur la motricité du sphincter anal externe ainsi qu'un effet thérapeutique dans les troubles de la continence urinaire vésico-urétral et/ou fécale.

La présente invention a en conséquence pour objet l'utilisation du modafinil pour la fabrication d'un médicament pour le traitement de l'incontinence urinaire et fécale et des troubles sphinctériens urétro-vésicaux et anaux.

Le médicament contenant le modafinil peut être présenté notamment sous forme convenant pour l'administration par voie orale. Généralement, les doses administrées peuvent être de 1 mg/kg à 100 mg/kg et de préférence de 5 à 100 mg/kg.

On donnera ci-après des résultats d'essais pharmacologiques, pharmaco-cliniques et cliniques mettant en évidence les effets du modafinil sur l'activité sphinctérienne ainsi que sur l'incontinence urinaire et/ou fécale d'origine neurologique.

**1) Etude de la réactivité sphinctérienne à l'aide de mesures urodynamiques de patients adultes et âgés ayant des troubles vésicosphinctériens liés à une insuffisance cervicosphinctérienne exclusive ou non.**

L'objectif de cette étude était d'étudier l'effet du modafinil administré en prise unique, en aveugle par rapport à du placebo, sur la réactivité sphinctérienne mesurée par le profil de pression urétrale chez des patients atteints de troubles vésicosphinctériens.

La méthode utilisée désignée "profil de pression urétral" ou encore nommée sphinctérométrie, consiste en l'étude des pressions urétrales enregistrées par l'intermédiaire d'un cathéter retiré progressivement depuis la vessie jusqu'au méat urétral. Cette méthode est décrite en détail dans A.R. Murphy, T.P. Stephenson, A.J. Wein "Urodynamics : principles, practice and application" Churchil Livingstone Ed. Edinburg 1984, 1 vol. 394 p; et Monographie SIFUD "Urodynamique et neuro-urologie" FIIS Ed. Paris, 1988, 1 vol. 256 p.

La méthode du profil urétral mesure la pression maximale urétrale (P.M.U.), la pression de clôture maximale qui est la différence entre la pression urétrale maximale et la pression régnant à l'intérieur de la vessie à un degré de remplissage vésical donné, et enfin la longueur totale du profil ou longueur fonctionnelle. L'appareil Electromed à eau a été utilisé.

Les mesures ont été effectuées sur vessie vide d'une part et vessie pleine d'autre part.

Les patients de sexe féminin ont reçu respectivement en prise unique soit 400 mg de modafinil, soit du placebo.

Le Tableau I donne les résultats relatifs à la pression maximale urétrale (P.M.U.) en cm d'eau.

**TABLEAU I**

| | MODAFINIL 400 mg (n = 24) | PLACEBO ( n = 24) |
|---|---|---|
| T0 (9h) | 36,6 ± 8,4 | 39,7 ± 9,6 |
| T1 (10 h) | 37,8 ± 10,1 | 39,9 ± 10,5 |
| T2 (11 h) | 40,1 ± 11,3 | 39,7 ± 10,8 |
| T3 (12 h) | 40,1 ± 10,5 | 38,3 ± 10,7 |

Le Tableau II donne les résultats relatifs à la pression de clôture maximale (en cm d'eau).

**TABLEAU II**

| | MODAFINIL 400 mg (n = 24) | PLACEBO (n = 24) |
|---|---|---|
| TO (9h) | 29,3 ± 9,0 | 32,3 ± 9,5 |
| T1 (10 h) | 30,0 ± 10,7 | 32,9 ± 9,9 |
| T2 (11 h) | 32,2 ± 12,3 | 32,9 ± 10,3 |
| T3 (12 h) | 32,9 ± 11,4 | 31,4 ± 10,2 |

Les résultats indiquent une augmentation significative de la pression de clôture maximale et de la pression maximale urétrale avec le modafinil par rapport au placebo qui n'a pas entrainé pour sa part de variation.

**2) Recherche en double aveugle de l'effet du modafinil dans l'incontinence urinaire d'origine neurologique**

Cette étude a été réalisée chez 40 sujets (âge moyen 10,77 ans) présentant une incontinence urinaire d'origine neurologique, ayant été ou non déjà traitée avec succès modeste (anticholinergiques).

Le modafinil a été prescrit à la posologie de 10 mg/kg (matin et soir) en double aveugle par rapport à du placebo.

Avant inclusion les patients ont été répartis en deux sous-groupes (réactifs et non-réactifs) en fonction de leur réponse à des tests pharmacologiques (détermination de la modification de pression de clôture vésicale après administration unique de 10 mg/kg de modafinil.

L'attribution des traitements a été équilibrée dans chacun de ces sous-groupes (10 traités et 10 témoins).

L'essai s'est déroulé sur 6 semaines, les deux premières sans traitement. L'instrument principal d'évaluation était un calendrier mictionnel rempli quotidiennement permettant de quantifier la continence nocturne, le nombre de périodes sèches par jour, le nombre de fuites par jour et par tranches horaires de deux heures.

Après avoir vérifié que les groupes Traité et Témoin étaient comparables lors des deux semaines de référence, nous avons pu montrer que le modafinil à la posologie de 10 mg/kg est capable d'améliorer à court terme, à la différence du placebo, la continence nocturne (cette différence disparaît après deux semaines) et à long terme (4 semaines) les fuites urinaires diurnes, surtout matinales.

Il est à noter que l'effet thérapeutique a été supérieur chez les patients réactifs au test pharmacologique d'inclusion. Cet effet "répondeur" a été démontré indépendant de l'effet "traitement". L'indépendance statistique entre l'effet "répondeur" et l'effet "traitement" permet de conclure à une réelle efficacité du modafinil.

**3) Etude des effets du modafinil sur la motricité du sphincter anal externe**

**Matériel et méthode**

**1 - Anesthésie**

Cette étude a été effectuée chez 6 chats des deux sexes d'un poids moyen de 2,5 kg anesthésiés au chloralose à la dose de 75 à 80 mg/kg. le rythme cardiaque et la pression artérielle sont enregistrés pendant toute la durée de l'expérimentation afin de détecter une éventuelle modification du niveau d'anesthésie.

**2 - Accès au sphincter anal externe**

Elle est effectuée par voie postérieure en pratiquant une incision de la peau au dessus et de part et d'autre de l'orifice anal. Les parties latérales et dorsale du sphincter sont séparées du tissu adipeux qui les entoure.

**3 - Dissection des nerfs honteux**

Ils sont situés latéralement par rapport au sphincter. Un nerf honteux est disséqué sur environ un centimètre puis sectionné, son bout central est alors posé sur des électrodes de stimulation incluses dans une gouttière en plexiglass.

**4 - Technique de stimulation**

Les stimulations sont effectuées sur le bout central d'un nerf honteux sectionné afin d'obtenir des réponses réflexes qui, seules, sont facilitées par la noradrénaline. Dans une autre expérience, le bout périphérique de ce nerf sectionné a été stimulé afin de stimuler les efférences sphinctériennes et obtenir une réponse directe. Les impulsions, fournies par un stimulateur (Grass S88), sont des chocs rectangulaires qui ont une durée de 0,1 ms; leur intensité varie en fonction de l'animal, mais est déterminée de telle sorte que l'amplitude des réponses soit inframaximale.

**5 - Technique de réception de l'activité électromyographique**

Pour enregistrer les réponses sphinctériennes à la stimulation du nerf honteux des dérivations bipolaires de l'activité électrique du sphincter anal externe sont effectuées au moyen d'électrodes implantées dans le muscle selon la technique de Basmadjian et Stecko (J. Appl. Physiol. 17, 749, 1962). La distance inter-électrode est la plus importante possible afin de recueillir des réponses qui représentent l'activité d'un grand nombre d'unités motrices. L'activité électrique, enregistrée grâce à un amplificateur (ECEM, amplificateur à liaison RC, bande passante 0.120 Hz, constante de temps 2s), est transmise à un convertisseur analogique numérique. Les signaux ainsi convertis sont traités par un ordinateur (MacIntosh) au moyen d'un logiciel approprié (MacLab). La réponse du sphincter anal externe, enregistrée correspond à la moyenne de 5 réponses successives.

**6 - Voies d'administration**

Le modafinil a été injecté par voie intraveineuse à la dose de 5 mg/kg, après dilution dans un solvant approprié.

**Résultats**

Il a été vérifié que l'injection intraveineuse du solvant, à dose identique à celle utilisée pour dissoudre le modafinil, n'induisait aucun effet sur les réponses du sphincter anal externe à la stimulation d'un nerf honteux.

Le modafinil, à la dose de 5 mg.kg i.v., entraîne une augmentation de l'amplitude de la réponse réflexe du sphincter anal externe. Les résultats sont donnés dans le Tableau III.

**TABLEAU III**

| Caractéristique des effets du modafinil sur la réponse réflexe du sphincter anal externe | | |
|---|---|---|
| Latence (mn) | Durée (mn) | Augmentation de l'amplitude de la réponse (n=3) |
| $10,00 \pm 5,00$ (n=3) | $18,70 \pm 6,00$ (n=3) | $76,70 \pm 20,00$ (n=3) |

L'injection intraveineuse de modafinil entraîne ainsi une facilitation du réflexe spinal sacré à l'origine de l'activité tonique du sphincter anal externe.

## Revendications

**1 -** Utilisation du modafinil et de ses isomères pour la fabrication d'un médicament destiné au traitement de l'incontinence urinaire et/ou fécale.

**2 -** Utilisation du modafinil et de ses isomères pour la fabrication d'un médicament destiné au traitement des troubles sphinctériens urétro-vésicaux.

**3 -** Utilisation du modafinil et de ses isomères pour la fabrication d'un médicament destiné au traitement des troubles sphinctériens anaux.

Office européen des brevets

# RAPPORT DE RECHERCHE EUROPEENNE

Numero de la demande
EP 93 40 2581

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| D,A | FR-A-2 385 693 (LAFON)<br>* le document en entier *<br>--- | 1-3 | A61K31/165 |
| A | EP-A-0 462 004 (LAFON)<br>* le document en entier *<br>----- | 1-3 | |

| DOMAINES TECHNIQUES RECHERCHES (Int.Cl.5) |
|---|
| A61K |

Le présent rapport a été établi pour toutes les revendications

| Lieu de la recherche | Date d'achèvement de la recherche | Examinateur |
|---|---|---|
| LA HAYE | 18 Novembre 1993 | KLAVER, T |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)